# EUROPÄISCHE PATENTSCHRIFT

(11) **EP 1 177 310 B1**
(45) Veröffentlichungstag und Bekanntmachung des Hinweises auf die Patenterteilung: **26.01.2005**
(21) Anmeldenummer: 00926778.2
(22) Anmeldetag: 30.03.2000
(51) Int. Cl.: C12P 13/04, C12P 1/04, C12N 1/20

(54) **VERFAHREN ZUR HERSTELLUNG VON L-PHOSPHINOTHRICIN DURCH ENZYMATISCHE TRANSAMINIERUNG MIT ASPARTAT**
METHOD FOR PRODUCING L-PHOSPHINOTHRICINE BY ENZYMATIC TRANSAMINATION WITH ASPARTATE
PROCEDE DE PREPARATION DE L-PHOSPHINOTHRICINE PAR TRANSAMINATION ENZYMATIQUE AVEC DE L'ASPARTATE

(30) Priorität: 30.04.1999 DE 19919848
(43) Veröffentlichungstag der Anmeldung: 06.02.2002
(73) Patentinhaber: Bayer CropScience GmbH, 65929 Frankfurt/Main (DE)
(72) Erfinder: BARTSCH, Klaus, D-61462 Königstein (DE)
(86) Internationale Anmeldenummer: PCT/EP2000/002809
(87) Internationale Veröffentlichungsnummer: WO 2000/066760

(56) Entgegenhaltungen:
- EP-A- 0 249 188
- EP-A- 0 349 965
- EP-A- 0 477 902
- BARTSCH K. ET AL.: "Stereospecific production of the herbicide Phosphothricin (Glufosinate): purification of Aspartate Transaminase from Bacillus stearothermophilus, cloning of the corresponding gene, aspC, and application in a coupled transaminase process" APPLIED AND ENVIRONMENTAL MICROBIOLOGY, Bd. 62, Nr. 10, Oktober 1996 (1996-10), Seiten 3794-3799, XP002144376
- Römpp-Lexikon Biotechnologie, Gentechnik, 1. Auflage (1992), Druckauflage 1999, S. 783 und S. 251
- SIRMAN T. ET AL: 'Extraction of organic acids using a supported liquid membrane' BIOCHEMICAL SOCIETY TRANSACTIONS Bd. 19, Nr. 3, 1991, Seite 274S
- OZADALI F. ET AL: 'Fed-batch fermentation with and without on-line extraction for propionic and acetic acid production by Propionibacterium acidipropionici' APPLIED MICROBIOLOGY AND BIOTECHNOLOGY Bd. 44, Nr. 6, 1996, Seiten 710 - 716

## Beschreibung

Die Erfindung betrifft das technische Gebiet der Synthese von Pflanzenschutzmittelwirkstoffen, insbesondere die Synthese von L-2-Amino-4-(hydroxymethylphosphinyl) buttersäure (L-Phosphinothricin, L-PPT) aus 4-(Hydroxymethylphosphinyl)-2-oxobuttersäure (HMPB, PPO) durch enzymatische Transaminierung in Gegenwart von Aspartat in Gegenwart einer PPO-spezifischen Aspartat-Transaminase (Asp-TA). Die Verbindung L-PPT, deren Salze und einige Derivate davon sind herbizid wirksame nicht-proteinogene Aminosäuren bzw. Salze und Derivate davon (DE-A-2717440). Die jeweilige L-Form ist dabei biologisch aktiv, während die jeweilige D-Form praktisch unwirksam ist (DE-A-2856260).

Es ist bereits bekannt, daß sich Transaminasen aufgrund ihrer hohen Stereoselektivität und einer relativ breiten Substratspezifität besonders für die chirale, enzymatische Synthese von Aminosäuren aus ihren korrespondierenden Ketosäurevorstufen eignen. Ein Nachteil für den technischen Einsatz von Transaminasen ist jedoch ihre Gleichgewichtskonstante von ca. 1, so daß das gewünschte Produkt im allgemeinen nur in einer 50 % igen Ausbeute entstehen kann (US-A-4,826,766). In EP-A-0344683 und in US-A-5,221,737 wird die Herstellung des herbiziden Wirkstoffs L-Phosphinothricin [(L-Homoalanin-4-yl-(methyl)phosphinsäure, L-2-Amino-4-(hydroxymethylphosphinyl)-buttersäure, L-PPT)], einer nicht-proteinogenen Aminosäure, durch Transaminierung aus der korrespondierenden Ketosäure [(2-Oxo-4-((Hydroxy)(Methyl)Phosphinoyl)-buttersäure, PPO)] mit 4-Aminobutyrat : 2-Ketoglutarat -Transaminase (GABA-Transaminase, EC 2.6.1.19) aus *Escherichia coli* beschrieben. Für eine quantitative Umsetzung wird ein hoher molarer Überschuß des Aminodonors Glutamat benötigt, was die Aufreinigung des Reaktionsproduktes erschwert.

Eine Lösung dieses Problems ist durch die Verwendung von Aspartat als Aminodonor möglich, da die korrespondierende Ketosäure Oxalacetat in wäßrigem Milieu instabil ist und spontan zu Pyruvat decarboxyliert, Durch die Entfernung eines Reaktionsproduktes aus dem Gleichgewicht kann keine Rückreaktion stattfinden und eine quantitative Umsetzung ist auch bei äquimolarem Einsatz von Ketosäure und Donoraminosäure möglich. Ein solches Verfahren wird z.B. in der EP-A-0135846 beschrieben.

Die Anwendung dieses Prinzips auf die enzymatische Synthese von L-Phosphinothricin war jedoch bisher nicht möglich, da die beschriebene GABA-Transaminase Aspartat als Aminodonor nicht akzeptiert und auch keine andere Transaminase mit gemeinsamer Spezifität für L-Phosphinothricin und Aspartat bekannt war.

Hilfsweise wurde ein gekoppeltes 2-Enzym-System, bestehend aus PPT-spezifischer Transaminase und Glutamat : Oxalacetat - Transaminase (GOT, EC 2.6.1.1) vorgeschlagen (EP-A-0249188 und EP-A-0477902). Bei dieser Reaktionsführung wird das bei der Synthese von L-PPT verbrauchte Glutamat mittels GOT aus Aspartat regeneriert. Die Aspartat-Transaminase selbst hat keine Spezifität für L-PPT/PPO. Die spontane Umwandlung von Oxalacetat zu Pyruvat führt auch für die Gesamtreaktion zu einer Gleichgewichtsverschiebung in Richtung L-PPT-Synthese. Dabei sind quantitative Produktausbeuten bei äquimolarem Einsatz von PPO und Aspartat und deutlichem Unterschuß von Glutamat möglich.

Durch diesen gekoppelten Enzymprozeß kann die Überdosierung der in der Substratlösung vorhandenen Donoraminosäuren gegenüber der Akzeptorketosäure PPO deutlich vermindert werden, was die Aufarbeitung der Produktlösung vereinfacht. Jedoch ist bei der gekoppelten Reaktionsführung weiterhin die Verwendung von Glutamat erforderlich, das - im Gleichgewicht mit Ketoglutarat - im Reaktionsprodukt verbleibt oder durch aufwendige Reinigungsverfahren von der strukturell sehr ähnlichen Aminosäure L-PPT abgetrennt werden muß. Außerdem ist die Optimierung der Reaktionsführung mit 2 Enzymen auf Grund der unterschiedlichen kinetischen Parameter schwieriger als mit einem Enzym.

Obwohl bisher bekannte Aspartat-Transaminasen, wie z.B. GOT keine Umsetzung von PPO zeigen, wurden nun überraschenderweise Aspartat-Transaminasen aus Mikroorganismen gefunden, die ebenfalls L-PPT/PPO mit hoher Spezifität als Substrat akzeptieren. Diese Enzyme katalysieren eine direkte Übertragung der alpha-Aminogruppe des Aspartats auf PPO.

Gegenstand der vorliegenden Erfindung ist daher ein Verfahren zur Herstellung von L-2-Amino-4-(hydroxymethylphosphinyl)-buttersäure (L-Phosphinothricin, L-PPT) der Formel (I), deren Derivaten und/oder Salzen, 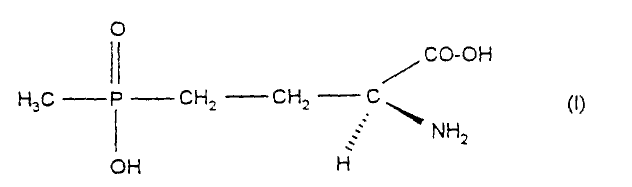 aus 4-(Hydroxymethylphosphinyl)-2-oxo-buttersäure (HMPB, PPO) der Formel (II), 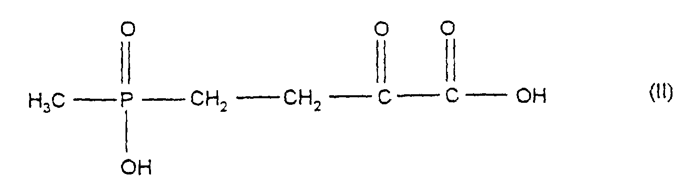 deren Derivaten und/oder Salzen als Akzeptor durch enzymatische Transaminierung in Gegenwart von Aspartat als Donor, wobei die Transaminierung in Gegenwart einer oder mehrerer akzeptor-spezifischen, vorzugsweise PPO-spezifischen Aspartat-Transaminasen (Asp-TA) zu Oxalacetat und der Verbindung der Formel (I), deren Derivate und/oder Salzen erfolgt, vorzugsweise in Gegenwart von einer oder mehreren thermostabilen und/oder isolierten Aspartat-Transaminase und ganz besonders bevorzugt in Gegenwart von einer oder mehreren Aspartat-Transaminasen mit einer möglichst geringen Substratspezifität für Pyruvat, so daß die Bildung des Nebenprodukts Alanin reduziert oder weitgehend vermieden werden kann.

Salze von L-PPT sind im allgemeinen Salze mit anorganischen und/oder organischen Säuren oder Mono- und Disalze mit anorganischen und/oder organischen Basen. Salze mit Säuren (Säureadditionssalze) sind beispielsweise Salze mit Mineralsäuren, wie Salzsäure (Hydrochlorid) oder Schwefelsäure (Sulfate), oder mit Kohlensäure (Carbonate, Hydrogencarbonate) oder mit organischen Säuren, wie Essigsäure (Acetate), Ameisensäure (Formiate), Propionsäure (Propiate) oder Weinsäure (Tartrate). Salze mit Basen sind beispielsweise Alkaliund Erdalkalimetallsalze, Ammoniumsalze, Salze mit organischen Aminen, wie primären, sekundären oder tertiären Aminen, und quartäre Ammoniumsalze.

Derivate sind beispielsweise Ester von L-PPT, die an der Phosphinsäuregruppe verestert sind, beispielsweise verestert sind mit C₁-C₁₂-Alkanolen wie Methanol, Ethanol, n-Propanol, i-Propanol, n-, i- und sec- oder tert-Butanol und C₃-C₆-Cycloalkanolen wie Cyclohexanol. Derivate sind auch Ester von L-PPT die alternativ oder zusätzlich an der Carbonsäuregruppe verestert sind, beispielsweise mit den bereits vorstehend genannten Alkoholen. Derivate sind auch das Carbonamid von L-PPT und dessen Derivate, gegebenenfalls N-Alkyl oder N,N-Dialkylamide mit vorzugsweise 1 bis 4 C-Atomen im Alkylteil.

Derivate von PPO sind beispielsweise deren Salze mit anorganischen und/oder organischen Basen, wobei dafür geeignete Basen bereits im Zusammenhang mit L-PPT genannt wurden. Derivate sind beispielsweise auch Ester von PPO, die an der Carbonsäuregruppe oder der Phosphinsäuregruppe oder beiden verestert sind. Als Alkohole für die Estergruppen eignen sich formal die für Ester von L-PPT geeigneten Alkohole, vorzugsweise die dort genannten Alkanole. Derivate sind auch das Carbonamid von PPO und dessen Derivaten, die an der Phosphinsäuregruppe verestert sind, sowie gegebenenfalls entsprechende N-Alkyl oder N,N-Dialkylamide.

Aspartat bezeichnet vorzugsweise L-Asparginsäure oder deren Salze, vorzugsweise Alkalimetallsalze. Als Aspartat kann aber auch L-Asparaginsäure in Mischungen mit D-Asparaginsäure, beispielsweise als racemische D,L-Asparaginsäure eingesetzt werden.

Alternativ kann in dem erfindungsgemäßen Verfahren gegebenenfalls vorhandenes Pyruvat aus der Reaktionsmischung physikalisch, chemisch und/oder enzymatisch entfernt werden, vorzugsweise durch Umsetzung mittels enzymatische Katalyse, z.B. durch Acetolactat-Synthase (ALS), Pyruvat-Decarboxylase, Pyruvat-Oxidase, insbesondere Acetolactat-Synthase; ganz besonders bevorzugt erfolgt die Umsetzung von Pyruvat in Gegenwart eines relativ thermostabilen Enzyms. Die so verwendeten Enzyme können gegebenenfalls in immobilisierter Form vorliegen.

Beide Substrate (Donor und Akzeptor) werden beispielsweise in einem molaren Verhältnis von 0,5-2 : 1 (bezogen auf L-Asparaginsäure : PPO) eingesetzt, vorzugsweise 0,75-1,5 : 1, insbesondere etwa äquimolar. Beim Einsatz von Gemischen von L- und D-Asparaginsäuren(salzen) ist die molare Menge von L-Asparaginsäure(salz) maßgebend. PPO-Derivate sind in molaren Mengen entsprechend PPO einzusetzen. Die Anwesenheit von Glutamat in der Substratlösung ist nicht notwendig. Einige der gefundenen Enzyme weisen eine ausgezeichnete Thermostabilität auf. Die Prozeßführung ist daher in einem weiten Temperaturbereich möglich, beispielsweise bei Temperaturen von 10 bis 95°C, vorzugsweise von 40 bis 90°C, insbesondere von 60 bis 85°C. Bei Enzymen, die keine besondere Thermostabilität aufweisen liegt der bevorzugte Temperaturbereich bei 20 bis 70°C, insbesondere bei 30 bis 40°C.

Durch die relativ hohen Temperaturen läßt sich die Reaktionsgeschwindigkeit erheblich beschleunigen, was auch die Umsetzung von konzentrierteren Substratlösungen (10 % ig) mit hohen Raum/Zeit-Ausbeuten ermöglicht. Die Reaktion erfolgt vorzugsweise bei einem pH-Wert im Bereich von 6,5-10, vorzugsweise von 7 bis 9, insbesondere von 7,5 bis 8,5 in einem entsprechend geeigneten Puffersystem mit einem pKa-Wert im Bereich von 7-9, unter anderem Phosphat- oder Tris-Puffer. Überraschenderweise besitzen die biochemisch näher charakterisierten Enzyme keine Spezifität für GABA und unterscheiden sich somit deutlich von bisher bekannten L-PPT/PPO-spezifischen Transaminasen.

Besonders hohe Konversionsraten lassen sich in der Reaktion erreichen, wenn die Entstehung von Alanin während der Transaminierung vermieden bzw. minimiert werden kann. Zu diesem Zweck können gegebenenfalls optimierte ASP-TA-Varianten ohne Substratspezifität für Pyruvat verwendet werden. Alternativ kann Pyruvat physikalisch, z. B. durch Verwendung selektiv permeabler Membranen und/oder chemisch bzw. enzymatisch, z. B. durch Umsetzung mit Pyruvat-Decarboxylase, Pyruvat-Oxidase oder Acetolactat-Synthase aus dem Reaktionsansatz entfernt werden (siehe z.B. Taylor et al., TIBTECH (1998) vol. 16, 412-418; Fotheringham et al., CHIMICA OGGI/chemistry today (1997), 9/10, 33-38; WO 98/53088).

Die Reinigung des Produkts, L-PPT, aus der Reaktionslösung kann gegebenenfalls nach bekannten und üblichen Verfahren erfolgen, beispielsweise durch Extraktion mit Methylisobutylketon oder über eine Kationenaustauscherchromatographie, z.B. mit Amberlite® IR 120 (Hersteller Sigma).

Das erfindungsgemäße Verfahren wird in den folgenden Beispielen weitergehend erläutert und die Erfindung in den Patentansprüchen definiert. Die nachfolgenden Beispiele sind insofern nicht limitierend zu verstehen.

### Beispiele:

### 1.) Isolierung von Bodenmikroorganismen mit L-PPT-spezifischer Aspartat-Transaminase Aktivität:

Je 1 g verschiedener Bodenproben (Humus, Lehm, Sand/Schwanheimer Düne, Frankfurt) wurden mit 10 ml 10 mM NaPhosphat-Puffer, pH = 7,0 für 1 h bei Raumtemperatur extrahiert. Aus den Extrakten wurden Anreicherungskulturen in folgendem Medium angeimpft:
5 mM Glucose
5 mM Succinat
10 mM Glycerin
10 mM PPO
10 mM L-Asparaginsäure
50 ml/l Lösung A
25 ml/l Lösung B

| | | | |
|---|---|---|---|
| Lösung A | 50 g/l K₂HPO₄ | | |
| Lösung B | 2,5 g/l MgSO₄ | | |
| | 0,5 g/l NaCl | | |
| | 25 ml/l aus einer Stammlösung | | |
| | enthaltend: | 1 g/l | FeSO₄ x 7 H₂O |
| | | 0,22 g/l | MnSO₄ x H₂ O |
| | | 0,1 g/l | H₃ BO₃ |
| | | 0,1 g/l | Na₂ MoO₄ x 2 H₂O |
| | | 0,18 g/l | ZnSO₄ x 7 H₂O |
| | | 0,16 g/l | CuSO₄ x 5 H₂O |
| | | 0,1 g/l | CoCl₂ x 6 H₂O |
| | | 1 ml/l | 1 N HCl |

Die Kulturen wurden für 3-5 Tage bei 28°C und 200 rpm auf einem Schüttler inkubiert. Aus einer der getesteten Bodenproben (Humus) ließen sich Mikroorganismen anreichern, die mit L-Asparaginsäure als alleiniger N-Quelle wachsen konnten. Die Kultur wurde mehrfach im gleichen Medium weiterpassagiert und dann zur Isolierung von Einzelklonen auf Agar-Medium der gleichen Zusammensetzung auspiattiert. Nach Inkubation für 3-5 Tage bei 28°C wurden insgesamt 100 Einzelkolonien isoliert und wieder in Flüssigmedium angeimpft (siehe oben). Die Vereinzelung auf Agar-Platten wurde noch 2 x wiederholt, um die Gewinnung von Reinkulturen sicherzustellen.
Nach diesen Selektionszyklen waren 20 Einzelstämme vorhanden, die mit L-Asparaginsäure als alleiniger N-Quelle wachsen konnten.

Zum Testen auf PPO/Asp-Transaminase-Aktivität wurden je 2 ml Kulturen der Stämme wie oben angezogen. Anschließend wurden je 400 µl der Kulturen mit 0,5 % Toluol, 0,5 % Ethanol für 30 min. bei 37°C permeabilisiert. Die Zellpellets wurden in je 50 µl Reaktionsmix bestehend aus 50 mM PPO, 50 mM L-Asparaginsäure, 50 mM Tris/HCl, pH = 8,0, 10 µM Pyridoxalphosphat resuspendiert und über Nacht bei 28°C inkubiert.

Zur qualitativen Bestimmung des gebildeten PPT wurden die Reaktionsüberstände 1:5 in Wasser verdünnt und davon je 5 µl durch Dünnschichtchromatographie auf HPTLC-Zellulose-Platten (Merck) mit n-Butanol : Eisessig : Wasser = 60 : 15 : 25 als Laufmittel analysiert. Die Aminosäuren wurden durch Ninhydrin-Färbung visualisiert. Bei 4 Stämmen (DSM 13353, DSM 13354, DSM 13355, DSM 13356; alle Stämme wurden bei der "Deutschen Sammlung von Mikroorganismen und Zellkulturen GmbH" hinterlegt.) konnte die Bildung von Phosphinothricin nachgewiesen werden. Die Enantiomerenreinheit des Reaktionsproduktes wurde durch chirale HPLC [mit der Trennsäule Chirex® (D) mit Penicillamin als Matrix (Hersteller Phenomenex) untersucht] (Laufmittel: 2 mM CuSO₄, 10 % Methanol, Flußrate: 0,5 ml/min., UV-Detektion: 254 nm, Retentionszeiten: L-PPT: ca. 17 min., D-PPT: ca. 21 min.). In allen 4 untersuchten Testproben konnte hierdurch L-PPT und kein D-PPT als Reaktionsprodukt nachgewiesen werden.

Zur Herstellung von L-PPT durch Biotransformation sowie einer quantitativen Analyse des Reaktionsverlaufs wurden je 1l Kulturen der Bodenbakterienstämme DSM 13354, DSM 13355 und DSM 13356 im Medium wie auf S. 7 beschrieben, für 48 Stunden bei 28°C angezogen. Die Zellen wurden durch Abzentrifugieren geerntet, 1x in 10 mM NaCl, 10 mM NaPhosphat, pH = 7,5 gewaschen und anschließend über Nacht lyophilisiert.
Zur Durchführung der Biotransformation wurden je 200 mg Zelltrockenmasse der oben bezeichneten Bodenbakterienstämme in 10 ml der folgenden Substratlösung resuspendiert:
100 mM PPO
200 mM L-Asparaginsäure
100 mM Tris/HCL, pH = 8,0
1 mM Pyridoxalphosphat
Die Ansätze wurden auf dem Inkubationsschüttler bei 200 rpm und 37°C inkubiert. Nach 1, 2, 4, 8, 24 und 30 h wurden je 200 µl Proben entnommen und in der HPLC, wie aus S. 8 beschrieben, analysiert. Die Meßergebnisse für L-PPT und L-Asparaginsäure sind in der Tabelle 1 zusammengefaßt. Die maximal erzielte Konversionsrate [produziertes L-PPT/PPO im Substrat x 100] lag bei ca. 59% (DSM 13355).

**Tabelle 1:**

| Reaktionsverlauf der PPO/Aspartat-Transaminierung durch Biotransformation mit Bodenisolaten | | | |
|---|---|---|---|
| Stamm | Reaktionszeit [h]* | L-PPT [mM] | Asparaginsäure [mM] |
| | | | |
| DSM 13354 | 1 | 3,9 | 174,0 |
| | 2 | 5,7 | 150,0 |
| | 4 | 10,3 | 100,0 |
| | 8 | 23,8 | 30,3 |
| | 24 | 38,3 | 0 |
| | 30 | 48,4 | 0 |
| | | | |
| DSM 13355 | 1 | 4,5 | 143,1 |
| | 2 | 7,7 | 122,7 |
| | 4 | 11,1 | 98,8 |
| | 8 | 24,8 | 76,4 |
| | 24 | 44,9 | 17,2 |
| | 30 | 59,1 | 9,8 |
| | | | |
| DSM 13356 | 1 | 5,7 | 138,1 |
| | 2 | 8,4 | 124,4 |
| | 4 | 12,5 | 95,9 |
| | 8 | 27,5 | 58,8 |
| | 24 | 51,3 | 14,3 |
| | 30 | 49,6 | 7,2 |

| | | | |
|---|---|---|---|
| *: Reaktionstemperatur: 37°C | | | |

### 2.) Nachweis der direkten PPO/Aspartat-Transaminierung mit Transaminase-Enzympräparaten:

Insgesamt 7 verschiedene, kommerziell erhältliche Transaminasen wurden auf PPO/Aspartat-Transaminierung getestet. Aus Mikroorganismen stammende (thermostabile Transaminasen AMN-001-01, -001-02, -001-03, -001-04, -001-05, enthalten in den Aminotransferase-Testkit von Diversa CAT# AMN-001 (1998); Glutamat-Oxalacetat-Transaminase (GOT), Glutamat-Pyruvat-Transaminase (GPT), Sigma). Die Enzympräparate wurden mit einer Proteinkonzentration von 5 mg/ml in 50 mM Tris/HCl-Puffer, pH = 8,0 gelöst und anschließend über Nacht bei 4°C gegen den gleichen Puffer dialysiert. Dadurch sollten in den Enzympräparationen möglicherweise vorhandene Aminodonoren und -akzeptoren, die als Zwischenüberträger bei der Transaminierung fungieren könnten, entfernt werden. Die Enzymlösungen wurden anschließend auf 1 mg/ml eingestellt und in 50 µl Ansätzen mit Reaktionspuffer bestehend aus 50 mM PPO, 50 mM L-Asparaginsäure, 50 mM Tris/HCl, pH = 8,0, 10 µM Pyridoxalphosphat für 1 h bei der für das jeweilige Enzym optimalen Temperatur inkubiert.

Die Enzymtests wurden durch Dünnschichtchromatographie und chirale HPLC, wie in Beispiel 1 beschrieben, analysiert. Bei 2 der thermostabilen Enzyme, AMN-001-03 und AMN-001-04 (Reaktionstemperatur: 80°C), konnte die enantioselektive Bildung von L-PPT durch Transaminierung aus L-Asparaginsäure nachgewiesen werden. Alle anderen getesteten Enzyme zeigten keine Reaktivität.

### 3.) Quantitative Untersuchung der PPO/Aspartat-Transaminierung mit der thermostabilen Transaminase AMN-001-03:

Aufgrund der höheren spezifischen Aktivität wurde die Transaminase AMN-001-03 für die genauere Charakterisierung der L-PPT-Synthese-Reaktion ausgewählt. 1 ml einer Substratlösung bestehend aus 40 mM PPO, 48 mM L-Asparaginsäure, 50 mM Tris/HCl, pH = 8,0, 0,1 mM Pyridoxalphosphat wurden mit 1 mg Transaminase AMN-001-03 bei 80°C inkubiert. Zur Analyse des Reaktionsverlaufs wurden über einen Zeitraum von 24 h je 50 µl Aliquots abgenommen und bei -20°C eingefroren. PPT und Aspartat wurden im Aminosäureanalysator (Biotronic LC 5001) bestimmt. Die Ergebnisse sind in Tabelle 2 dargestellt. Unter den gewählten Bedingungen wurde das Reaktionsgleichgewicht der L-PPT-Synthese nach 2-4 h erreicht. Der eingesetzte Aminodonor L-Asparaginsäure war nach 7 h vollständig verbraucht. Es wurde eine Konversionsrate [produziertes L-PPT/PPO im Substrat x 100] von ca. 75 % erzielt.

**Tabelle 2:**

| Reaktionsverlauf der PPO/Aspartat-Transaminierung mit Transaminase AMN-001-03 | | |
|---|---|---|
| Reaktionszeit [h]* | L-PPT [mM] | Aspartat [mM] |
| | | |
| 0 | 0 | 53,4 |
| 1 | 9,5 | 47,8 |
| 2 | 20,8 | 33,8 |
| 4 | 25,7 | 12,5 |
| 7 | 29,7 | 0 |
| 24 | 28,1 | 0,4 |

| | | |
|---|---|---|
| *: Reaktionstemperatur: 80°C | | |

### 4.) Enzymatische, chirale Synthese von L-PPT aus PPO und Aspartat mit partiell gereinigter thermostabiler Transaminase AMN-001-03:

Für die Syntheseversuche wurde partiell gereinigte Transaminase AMN-001-03 mit einer spezifischen Aktivität von 107 nkat/mg Protein (1 nkat = 1 nmol Aspartat/sec.) eingesetzt. Die Reaktionslösung mit einem Volumen von 1 ml enthielt 552 mM PPO (10 %), 700 mM L-Asparaginsäure, 0,1 mM Pyridoxalphosphat, pH = 8,0, eingestellt mit KHCO₃ und 11,5 mg Enzym. Der Ansatz wurde bei 80°C inkubiert. Probenabnahme und Analytik erfolgten wie in Beispiel 3 beschrieben.

Die Ergebnisse sind in Tabelle 3 zusammengefaßt. In diesem Versuch war das Reaktionsgleichgewicht bereits nach 1 h erreicht. Der Aminodonor L-Asparaginsäure war nach 4 h nahezu vollständig verbraucht. Die Konversionsrate betrug ca. 52 % und die Raum/Zeit-Ausbeute lag bei 4,5 [g L-PPT/g Biokatalysator/ h]. In einem Parallelversuch mit gleicher Substratlösung und Enzymkonzentration aber einer Reaktionstemperatur von 60°C, wurde eine ähnliche Konversionsrate bei allerdings deutlich verminderter Reaktionsgeschwindigkeit erreicht. Die Raum/Zeit-Ausbeute betrug nur 0,95 [g L-PPT/g Biokatalysator/h]. Diese Ergebnisse belegen die große Bedeutung der hohen Temperaturstabilität der Transaminase für die Umsatzrate und eine effektive Reaktionsführung.

Die nur mäßige Konversionsrate von 52 % ist hauptsächlich auf die Bildung des Nebenproduktes Alanin durch Transaminierung von Pyruvat zurückzuführen. Wesentlich höhere Konversionsraten lassen sich erreichen, wenn die Entstehung von Alanin während der Reaktion vermieden wird.

**Tabelle 3:**

| Herstellung von L-PPT durch Transaminierung mit partiell gereinigter thermostabiler Transaminase AMN-001-03 | | | |
|---|---|---|---|
| Reaktionszeit [h]* | L-PPT [mM] | Aspartat [mM] | Alanin [mM] |
| | | | |
| 0 | 0 | 700,0 | 0 |
| 0,5 | 155,3 | 405,8 | 0 |
| 1 | 286,4 | 193,1 | 98,7 |
| 2 | 288,5 | 15,2 | 181,5 |
| 4 | 284,0 | 1,9 | 284,1 |
| 8 | 251,9 | 1,3 | 234,5 |

| | | | |
|---|---|---|---|
| *: Reaktionstemperatur: 80°C | | | |

## Patentansprüche

1. Verfahren zur Herstellung von L-2-Amino-4-(hydroxymethylphosphinyl)-buttersäure (L-Phosphinothricin, L-PPT) der Formel (I), deren Derivaten, welche aus der Gruppe der Carbonsäureester und -amide und Phosphinsäureester ausgewählt sind, und/oder deren jeweiligen Salzen 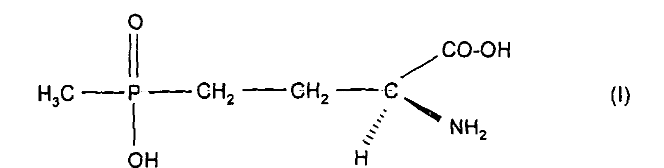 aus 4-(Hydroxymethylphosphinyl)-2-oxo-buttersäure (HMPB, PPO) der Formel (II), 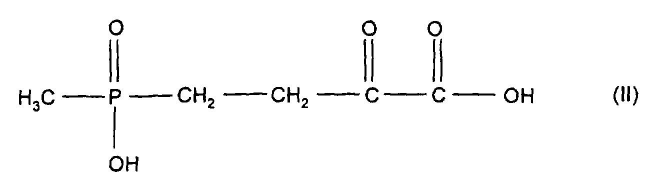 deren Derivaten, welche aus der Gruppe der Carbonsäureester und - amide und Phosphinsäureester ausgewählt sind, und/oder deren jeweiligen Salzen als Akzeptor durch enzymatische Transaminierung in Gegenwart von Aspartat als Donor, wobei die Transaminierung in Gegenwart einer oder mehrerer thermostabilen, akzeptor-spezifischen, Aspartat-Transaminasen (Asp-TA) zu Oxalacetat und der Verbindung der Formel (I), deren Derivaten und/oder Salzen erfolgt.

2. Verfahren nach Anspruch 1, wobei die Transaminierung bei einer Reaktionstemperatur von 80°C durchgeführt wird.

3. Verfahren nach Anspruch 1 oder 2, **dadurch gekennzeichnet, daß** vorhandenes Pyruvat aus der Reaktionsmischung physikalisch, chemisch und/oder enzymatisch entfernt wird.

## Claims

1. A process for preparing L-2-amino-4-(hydroxymethylphosphinyl)butyric acid (L-phosphinothricin, L-PPT) of the formula (I), its derivatives which are selected from the group of carboxylic esters and carboxamides and phosphinic esters and/or its respective salts 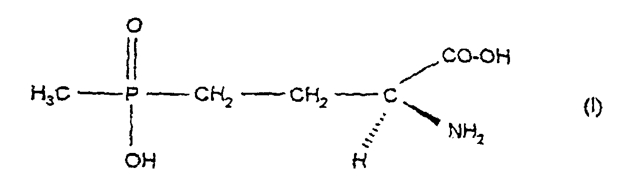 from 4-(hydroxymethylphosphinyl)-2-oxobutyric acid (HMPB, PPO) of the formula (II) 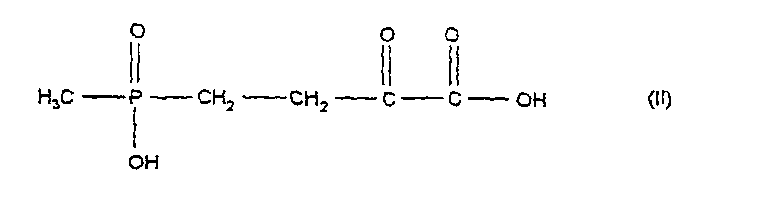 its derivatives which are selected from the group of carboxylic esters and carboxamides and phosphinic esters and/or its respective salts as acceptor by enzymatic transamination in the presence of aspartate as donor, where the transamination takes place in the presence of one or more thermally stable acceptor-specific aspartate transaminases (Asp-TA) to give oxaloacetate and the compound of the formula (I), its derivatives and/or salts.

2. The process as claimed in claim 1, wherein the transamination is carried out at a reaction temperature of 80°C.

3. The process as claimed in claim 1 or 2, wherein pyruvate which is present is removed from the reaction mixture by physical, chemical and/or enzymatic means.

## Revendications

1. Procédé pour la préparation d'acide L-2-amino-4-(hydroxyméthylphosphinyl)-butyrique (L-phosphinothricine, L-PPT) de formule (I), de ses dérivés, qui sont pris dans le groupe des esters et amides d'acides carboxyliques et des esters de l'acide phosphinique et/ou leurs sels correspondants 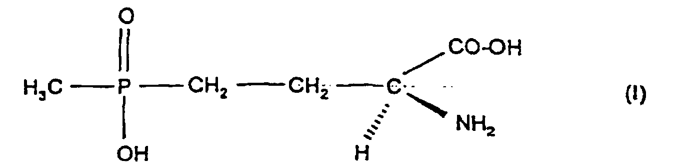 à partir d'acide 4-(hydroxyméthylphosphinyl)-2-oxobutyrique (HMPB, PPO) de formule (II) 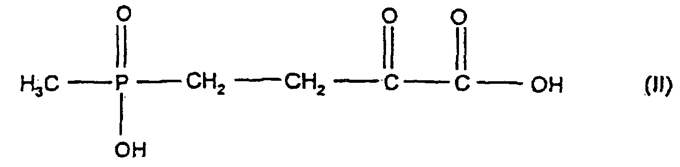 ses dérivés, qui sont pris dans le groupe des esters et amides d'acides carboxyliques et des esters de l'acide phosphinique et/ou leurs sels correspondants en tant qu'accepteurs, par transamination enzymatique en présence d'aspartate en tant que donneur, en présence d'une ou plusieurs aspartate-transaminases (Asp-TA) spécifiques d'accepteur, stables à la chaleur, en oxalacétate et le composé de formule (I), ses dérivés et/ou sels.

2. Procédé selon la revendication 1, où la transamination est mise en oeuvre à une température réactionnelle de 80°C.

3. Procédé selon la revendication 1 ou 2, **caractérisé en ce qu'**on élimine le pyruvate présent du mélange réactionnel par des voies physiques, chimiques et/ou enzymatiques.
